# FASCICULE DE BREVET EUROPEEN

(11) **EP 1 390 346 B1**
(45) Date de publication et mention de la délivrance du brevet: **09.04.2008**
(21) Numéro de dépôt: 02732843.4
(22) Date de dépôt: 30.04.2002
(51) Int. Cl.: C07C 409/00

(54) **UTILISATION D'UN STABILISATEUR THERMIQUE DE PEROXYDES ORGANIQUES**
VERWENDUNG EINES THERMISCHEN STABILISATORS FÜR ORGANISCHE PEROXIDE
USE OF AN ORGANIC PEROXIDE HEAT STABILISER

(30) Priorité: 17.05.2001 FR 0106526
(43) Date de publication de la demande: 25.02.2004
(73) Titulaire: ARKEMA FRANCE, 92700 Colombes (FR)
(72) Inventeur: GRIMALDI, Sandra, F-69110 Sainte Foy-les-Lyon (FR); GUERRET, Olivier, F-64230 Mazerol (FR); COUTURIER, Jean-Luc, F-69006 Lyon (FR); DEBAUD, Fabien, F-69005 Lyon (FR)
(74) Mandataire: Bonnel, Claudine
(86) Numéro de dépôt international: PCT/FR2002/001500
(87) Numéro de publication internationale: WO 2002/092561

(56) Documents cités:
- EP-A- 0 436 314
- FR-A- 2 792 321
- NL-A- 7 401 405
- US-A- 3 702 869
- US-A- 5 739 096
- DATABASE WPI Section Ch, Week 199033 Derwent Publications Ltd., London, GB; Class A17, AN 1990-246880 XP002188448 & AU 42933 89 A (ICI AUST OPERATIONS), 26 avril 1990 (1990-04-26)
- DATABASE WPI Section Ch, Week 200026 Derwent Publications Ltd., London, GB; Class A12, AN 2000-298623 XP002188449 & JP 2000 086816 A (YOKOHAMA RUBBER CO LTD), 28 mars 2000 (2000-03-28)

## Description

La présente invention concerne l'utilisation d'un stabilisateur thermique de peroxydes organiques.

L'utilisation d'un solvant, miscible avec un peroxyde d'alkyl(inférieur)cétone et présentant un point d'ébullition compris entre environ 185 et 225°C, pour stabiliser thermiquement le peroxyde organique a été divulguée dans le document FR 1594180. Ce document enseigne que le solvant est choisi parmi les esters, les aldéhydes, les cétones, les hydrocarbures, les hydrocarbures halogénés et les époxydes. Il enseigne également qu'en plus du solvant désensibilisant thermique, des stabilisants pyrrolidiniques peuvent être utilisés pour rendre les peroxydes de cétone stables au stockage.

La demande française FR 2792321 décrit un procédé de fabrication d'une résine à rhéologie contrôlée d'un homopropylène ou d'un copolymère du propylène dans lequel, à la place des peroxydes de l'état de la technique ou en plus de ceux-ci, des radicaux libres stables tels que les nitroxydes sont incorporés dans la résine à modifier, placée par exemple dans une zone fondue d'une extrudeuse. Le document JP 2000-86816 concerne une composition de caoutchouc contenant en plus du caoutchouc, un sel métallique d'acide carboxylique insaturé, un peroxyde organique et un radical nitroxyde à groupement polaire, ce dernier améliorant la dispersité du sel métallique d'acide carboxylique insaturé dans le caoutchouc.

Les radicaux nitroxyle sont des espèces radicalaires très stables possédant une fonction N-O. On peut se reporter à la publication de D. Griller et K. Ingold dans Accounts of Chemical Research, 1976, 9, 13-19 ou à celle de A. Forrester et coll. « Organic Chemistry of Stable Free Radicals » dans Academic Press, 1968 pour la définition.

Ces radicaux ont la faculté de réagir très rapidement avec les radicaux carbonés issus de la décomposition d'initiateurs ou issus de processus radicalaires, tels que les polymérisations radicalaires. Cette réactivité a été mise à profit en incorporant ces radicaux nitroxyle dans des processus de polymérisation radicalaire, tels que la polymérisation du styrène ou des acrylates, des processus de copolymérisation, de dégradation et de modification du polypropylène.

La demanderesse vient de constater que les radicaux nitroxyles peuvent stabiliser thermiquement les peroxydes et permettent éventuellement d'accroître la stabilité de ceux-ci au stockage.

L'objet de la présente invention est l'utilisation d' un stabilisateur thermique de peroxydes organiques caractérisé en ce qu'il renferme au moins un radical nitroxyle dans sa molécule. De préférence, le stabilisateur thermique de peroxydes organiques est un composé N-hétérocyclique avec l'atome d'azote du radical nitroxyle faisant partie du cycle.

Le document JP 2000-86816 concerne une composition de caoutchouc contenant en plus du caoutchouc, un sel métallique d'acide carbocyclique insaturé, un peroxyde organique et un radical nitroxyde à groupement polaire, ce dernier améliorant la dispersivité du sel métallique d'acide carbocyclique insaturé dans le caoutchouc.

Comme stabilisateur thermique, on peut citer notamment les composés représentés par les formules I , II et III 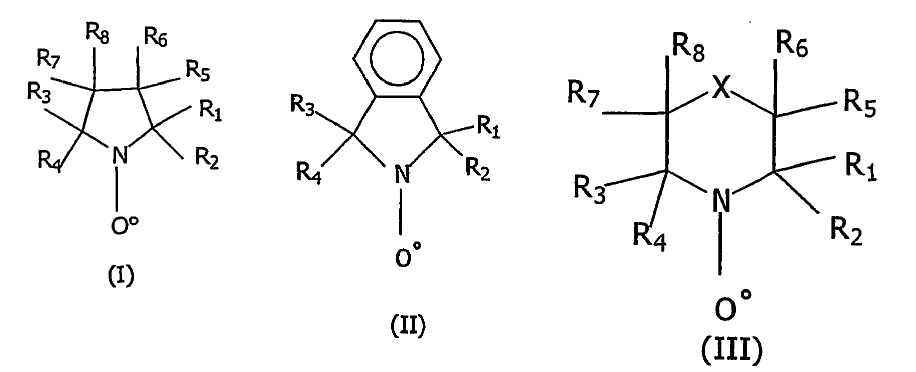 dans lesquelles R₁, R₂, R₃ et R₄ pouvant être identiques ou différents représentent un atome d'hydrogène, un atome d'halogène tel que le fluor, le chlore, le brome ou l'iode, un groupement hydrocarboné linéaire, ramifié ou cyclique, saturé ou insaturé tels qu'un radical alkyle ou phényle, une chaîne de polymère pouvant par exemple être une chaîne de poly(meth)acrylate d'alkyle comme le polyméthacrylate de méthyle, de polydiène, de polyoléfine ou de polystyrène, un groupement fonctionnalisé tel qu'un groupement cyano -CN, un groupement ester-COOR, un groupement amide -CON(R)₂, un groupement alcoxyle-OR, un groupement phosphonate -PO(OR)₂ dans lequel R représente une chaîne hydrocarbonée ayant de 1 à 9 atomes de carbone et dans lesquelles R₅, R₆, R₇, R₈, pouvant être identiques ou différents, peuvent être choisis dans la même famille de groupement que celle qui vient d'être envisagée pour R₁, R₂, R₃, R₄ et de plus, peuvent représenter un groupement hydroxyle -OH, un groupement acide tel que -COOH ou -PO(OH)₂ ou -SO₃H De plus, X dans la formule ( III ) représente un groupement bivalent choisi parmi le méthylène -CH₂ - , le -C(OR°)(OR')-, le carbonyl - C(O)-, l'oxy -O- et -CHZ- avec Z représentant un reste monovalent choisi parmi les groupements cyano : -CN, hydroxyle: -OH, amino : -NR°R', alcoxy : -OR°, iminoyl - N=CR°R', carboxylate : -OC(O)-R°, amide : -NHR°-C(O)R', dans lesquels R° et R' identiques ou différents représentent un atome d'hydrogène, un groupement alkyle, linéaire ou ramifié ayant un nombre d'atomes de carbone allant de 1 à 20, un groupement benzyle ou phényle. X dans la formule (III) peut également représenter un groupement phosphonate : -OP(O)R"R'"- avec R" et R''' ayant la même signification que Z.

Peuvent également convenir comme stabilisateur thermique, les composés représentés par la formule générale ( IV ) 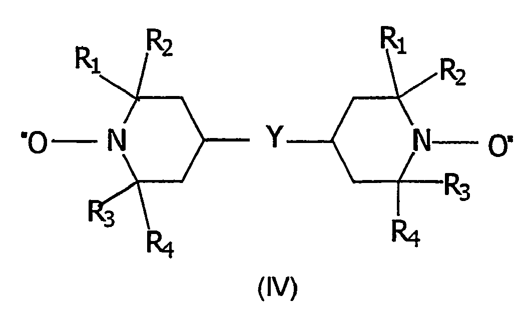 dans laquelle R₁, R₂, R₃, R₄, pouvant être identiques ou différents ont la même signification que ceux utilisés pour les formules (I) à (III) et Y représente un groupement bivalent choisi parmi :

--OC(O)-(CRₐR_{b})ₙ-C(O)O- ,

--NH-( CRₐR_{b})ₙ NH-- ,

--NHC(O)-( CRₐR_{b})ₙ -C(O)NH--,

-- S -- , --O--;

Rₐ et R_{b} , identiques ou différents, représentent un atome d'hydrogène ou un radical alkyle, linéaire ou ramifié, ayant un nombre d'atomes de carbone allant de 1 à 10 et n représente un nombre entier allant de 0 à 20 .

Selon l'invention, en plus des composés de formule générale (IV), d'autres composés renfermant plusieurs radicaux nitroxyles, comme par exemple ceux représentés par la formule générale (V) peuvent également convenir comme stabilisateur thermique: 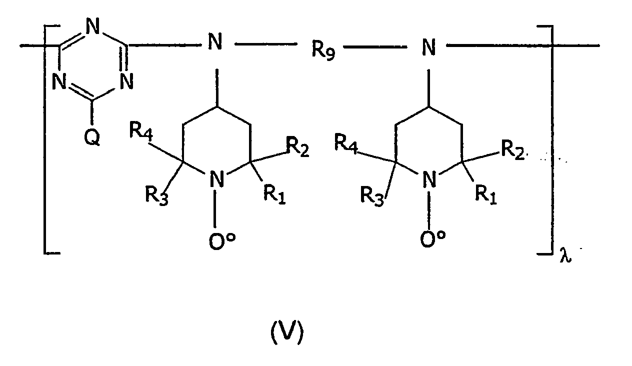 dans laquelle R₁, R₂, R₃ et R₄ pouvant être identiques ou différents, ont la même signification que ceux des formules précédentes ( I ) à (IV), □ est un nombre entier compris entre 1 et 20, R₉ représente un groupement alkylène ayant un nombre d'atomes de carbone allant de 2 à 12 qui peut être interrompu par un -O-ou - NR₁₀- avec R₁₀ désignant un atome d'hydrogène, un groupement alkyle ayant un nombre d'atomes de carbone compris entre 1 et 12, ou un groupement cycloalkyle et Q représente un radical -OR₁₁, -NHR₁₂, ou -NR₁₂R₁₃ où R₁₁ représente un radical alkyle, linéaire ou ramifié ayant un nombre d'atomes de carbone allant de 1 à 12, un radical alcoxyalkyle en C₃- C₁₂, un radical cyclohexyle, un radical benzyle, un radical phényle, un radical tolyle ou un reste 2, 2, 6, 6-tétrapipéridinyle, R₁₂ et R₁₃ ont la même signification que R₁₁, et de plus, peuvent également former ensemble et avec l'atome d'azote qui les porte un radical hétérocyclique à 5, 6 ou 7 chaînons pouvant contenir en outre un oxygène.

Les composés de formule générale (V) le plus souvent utilisés sont ceux obtenus par oxydation des amines commercialisées par la Société CIBA sous la dénomination Chimasorb 944 où R₁, R₂, R₃ et R₄ désignant chacun un groupement méthyle, R₉ un groupement alkylène à 6 atomes de carbone, Q représentant un radical -N(O°)-C₈H₁₁ et λ étant un nombre entier compris entre 2 et 4.

Les composés de formule générale (III) préférés sont ceux pour lesquels R₁, R₂, R₃ et R₄ désignent chacun un groupement méthyle, R₅, R₆, R₇ et R₈ représentent chacun un atome d'hydrogène et X un groupement -CHZ-.

En particulier, on peut citer comme composés de formule générale (III) le 2,2,6,6-tétraméthyl-1-pipéridinyloxy généralement commercialisé sous la dénomination TEMPO, le 4-hydroxy 2,2,6,6-tétraméthyl-1-pipéridinyloxy commercialisé sous la dénomination 4-hydroxy TEMPO, le 4-méthoxy-2,2,6,6-tétraméthyl-1-pipéridinyloxy communément appelé 4-méthoxy-TEMPO, le 4-oxo-2,2,6,6 tétraméthyl-1-pipéridinyloxy communément appelé 4-oxo-TEMPO.

Les composés de formule générale (III) particulièrement préférés sont ceux représentés par la formule suivante: 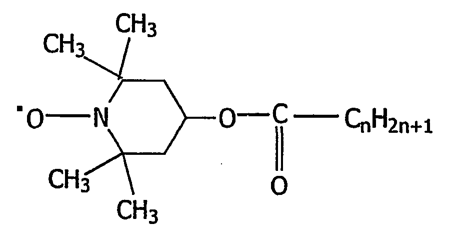 avec n pouvant varier de 1 à 20.

Les composés tels que le 2,2,5,5 tétraméthyl-1-pyrrolidinyloxy commercialisé sous la marque PROXYL, le 1-pipéridinyloxy-2,2,6,6-tétraméthyl- 4 - [ ( 1-oxooctadecyl ) oxy], le 1-pipéridinyloxy-4,4'-((1,10-décanediyl)bis(oxy)bis(2,2,6,6 - tétraméthyl) nitroxyde commercialisé sous la marque CXA 5415 par la Société Ciba Specialty Chemical, le 2,2,6,6-tétraméthyl-4-hydroxypipéridine-1-oxyl monophosphonate, le 3-carboxy-2,2,5,5-tétraméthylpirrolidinyloxy (communément appelé 3-carboxy Proxyl ) sont également préférés.

Les composés non cycliques tels que ceux représentés par la formule générale **( VI )** peuvent également convenir comme stabilisateurs thermiques: 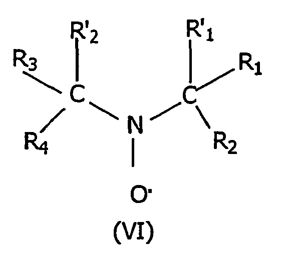 dans laquelle R₁, R₂, R₃, R₄, R'₁ et R'₂ pouvant être identiques ou différents ont la même signification que R₁, R₂, R₃, R₄ des formules générales précédentes.

La quantité de stabilisateur thermique présent dans la composition de peroxyde organique stable, selon l'invention, est de préférence comprise entre 0,1 et 99 % en poids et avantageusement comprise entre 0,2 et 30 % .

Comme peroxydes organiques, on peut citer notamment les peroxydes de dialkyle, les hydroperoxydes ainsi que leurs sels, les peroxycétals et les peroxyesters. Les peroxydes de dialkyle, en particulier les peroxydes de dialkyle cycliques dérivant de l'oxydation des cétones, les peroxycétals et les hydroperoxydes sont particulièrement préférés.

A titre d'exemple de peroxydes de dialkyle, on peut citer le 2,5-diméthyl-2,5-di(tert-butylperoxy) hexyne-3, le ditert-butyl peroxyde, le ditert-amyl peroxyde, le tert-butyl cumyl peroxyde, le di(tert-butylperoxy-isopropyl)-benzène, le 2,5-diméthyl-2,5-di(tert-butylperoxy) hexane, le dicumyl peroxyde , 3,6,9 - triéthyl- 3,6,9 triméthyl - 1,4,7 - triperoxonane .

A titre d'exemple de peroxyesters, on peut citer le tert-butyl peroxybenzoate, le tert-butyl peroxyacétate, le tert-butyl peroxy-3,5,5-triméthylhexanoate, le 2,5-diméthyl-2,5-di(benzoylperoxy) hexane, le OO-tert-butyl-O-(2-éthylhexyl) monoperoxycarbonate, le OO-tert-butyl-O-isopropyl-monoperoxycarbonate , le OO-tert-amyl-O-(2-ethylhexyl) monoperoxycarbonate, le tert-butylperoxy isobutyrate, le tert-butyl peroxy-2-éthylhexanoate, le tert-butyl peroxy-2-éthylhexanoate, le tert-amyl peroxy-2-éthylhexanoate (Luperox 575), le 2,5-diméthyl-2,5-di(2-éthylhexanoylperoxy) hexane (Luperox 256), le 3,6,9-triéthyl-1,4,7-triperoxonane, le tert-butyl peroxypivalate (Luperox 11 M75), le tert-amyl peroxypivalate, le tert-butyl peroxynéodécanoate, le tert-amyl peroxynéodécanoate, l'□-cumyl peroxynéodécanaote, le 3-hydroxy-1,1-diméthylbutyl peroxynéodécanoate

En tant qu'hydroperoxydes, on peut citer l'hydroperoxyde de tert-butyle, l'hydroperoxyde de tert-amyle, l'hydroperoxyde de cumyle, le 2,5-diméthyl-2,5-di(hydroperoxy) hexane, le monohydroperoxyde de diisopropylbenzène, le dihydroperoxyde de diisopropylbenzène, l'hydroperoxyde de paramenthyle.

En tant que sels d'hydroperoxydes, on peut citer le sel disodique de 1,3- et 1,4-bis (2-hydroperoxydeprop-2-yl) benzène.

En tant que peroxycétals, on peut citer l'éthyl-3,3-di(tert-butylperoxy) butyrate, l'éthyl-3,3-di(tert-amylperoxy) butyrate, le n-butyl-4,4-di(tert-butylperoxy) valérate, le 2,2-di(tert-butylperoxy) butane, le 1,1-di(tert-butylperoxy) cyclohexane, le 1,1-di(tert-butylperoxy)-3,5,5-triméthyl cyclohexane, le 1,1-di(tert-amylperoxy) cyclohexane.

Les peroxyesters tels que , Luperox 575 (tert-amyl peroxy-2-éthylhexanoate), Luperox 256 (le 2,5-diméthyl-2,5-di(2-éthylhexanoylperoxy) hexane )et Luperox 11 M75 (le tert-butyl peroxypivalate) nécessitant une température de stockage inférieure à la température ambiante sont avantageusemant choisis.

### PARTIE EXPERIMENTALE

Luperox 101 = 2,5-diméthyl-2,5-di (tert-butylperoxy) hexane
Luperox 802 = 1,4-bis (tert-butylperoxyisopropyl) benzène
Luperox 331 M50 = 1,1-di (tert-butylperoxy)
Luperox TBH70X* = hydroperoxyde de tertio-butyle
TEMPO : 2,2,6,6-tétraméthyl-1-pipéridinyloxy
AF845 : 1-pipéridinyloxy-2,2,6,6-tétraméthyl-4-[(1-oxooctadecyl)oxy]
CXA 5415 : 1-pipéridinyloxy-4,4'-((1,10-décanediyl)bis(oxy)bis(2,2,6,6 - tétraméthyl) nitroxyde
*solution à 14 % dans l'isododécane

Le Tableau I illustre l'effet bénéfique des stabilisateurs ( TEMPO , AF845 et CXA 5415 ) sur la température de décomposition des différents peroxydes organiques.

**TABLEAU I**

| Peroxyde organique ou mélange | Ratio massique Peroxyde / stabilisateur | Température de début de décomposition (°C) |
|---|---|---|
| Luperox 101 | ----- | 120 |
| Luperox 101 + TEMPO | 10 | 125 |
| Luperox 101 + AF 845 | 3 | 130 |
| Luperox 802 | ----- | 120 |
| Luperox 802 + TEMPO | 12 | 130 |
| Luperox 802 + AF 845 | 4 | 130 |
| Luperox 802 + CXA 5415 | 4 | 130 |
| Luperox 331 M50 | ----- | 100 |
| Luperox 331 M50 + TEMPO | 5 | 115 |
| Luperox TBH70X | ----- | 190 |
| Luperox TBH70X + TEMPO | 5 | 205 |

### Produits utilisés pour la dégradation

- Polypropylène de MFI = 3,3 (230°C / 2,16kg)
- Peroxyde organique : 2,5-diméthyl-2,5-di(tertiobutylperoxy)hexane - Luperox 101 (teneur en peroxyde 95%)
- Stabilisateur : TEMPO

### Procédure expérimentale

La dégradation du polypropylène est effectuée dans une extrudeuse bi-vis corotative de type BRABENDER. Cette extrudeuse comporte 4 zones Z1 à Z4 dont les températures respectives sont 200°C / 220 °C / 220°C / 180 °C. Le débit d'extrusion en sortie de zone Z4 est fonction de la vitesse de vis, il varie typiquement de 4,5 à 5 kg/h. Le jonc est granulé après refroidissement. Le produit est caractérisé par la valeur de MFI (mesurée à 230°C - 2,16kg), par la valeur du module ( mesurée suivant la norme ISO 178 ) et par la valeur de l'essai de choc Charpy ( mesurée à 23 ° C suivant la norme ISO 179/1eA ).

Le polypropylène est réduit sous forme de poudre sur laquelle une solution à base d'acétone, de peroxyde organique et de TEMPO est adsorbée. Après adsorption, l'acétone est évaporée et le mélange réactif à base de polypropylène est introduit par une trémie en zone 1 (Z1).

Le Tableau II résume les résultats obtenus en dégradation du problème avec une composition stabilisante selon l'invention ( exemples 3 et 4 effectués à partir de compositions de peroxyde organique et de TEMPO alors que l'exemple 5 a été conduit en dispersant le peroxyde et le TEMPO séparément sur la poudre de polypropylène ).

| Exemple | Peroxyde (ppm) | TEMPO (ppm) | Rapport molaire TEMPO / Peroxyde | MFI (dg/min) | Module (MPa) | Choc charpy Entaillé Energie à 23°C (kJ/m²) |
|---|---|---|---|---|---|---|
| 1 | 0 | 0 | - | 3,3 | 1270 | 3,1 |
| 2 | 500 | 0 | 0,0 | 33,4 | 1170 | 2,3 |
| 3 | 500* | 25* | 0,1 | 25,8 | 1170 | 2,6 |
| 4 | 2000* | 1020* | 1,0 | 23,6 | 1180 | 2,7 |
| 5 | 2000 | 1020 | 1,0 | 23,5 | 1180 | 2,7 |

| | | | | | | |
|---|---|---|---|---|---|---|
| *Utilisation d'une composition (pré-mélange) à base de peroxyde organique et de TEMPO - TABLEAU II | | | | | | |

L'incorporation d'une composition à base de peroxyde organique et de nitroxyde tel que le TEMPO, permet d'obtenir un polypropylène avec un indice de fluidité plus faible, avec une meilleure propriété choc et en conservant la même rigidité. La comparaison des exemples 4 et 5 montre clairement que l'utilisation d'un pré-mélange conduit à des propriétés identiques à celles obtenues à partir des constituants utilisés séparément.

## Revendications

1. Utilisation d'un composé stabilisateur thermique N-hétérocyclique renfermant au moins un radical nitroxyle avec l'atome d'azote du radical nitroxyle faisant partie du cycle pour préparer une solution thermiquement stable de peroxyde organique, la température de début de décomposition de ladite solution comprenant le stabilisateur étant > à la température de début de décomposition de la solution ne comprenant pas le stabilisateur.

2. Utilisation selon la revendication 1 **caractérisée en ce que** le stabilisateur thermique est représenté par au moins une des formules (I) , (II) , (III), (IV), (V) et (VI) suivantes : 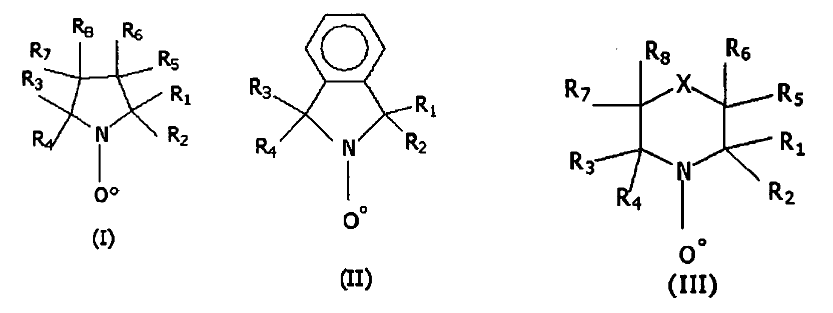 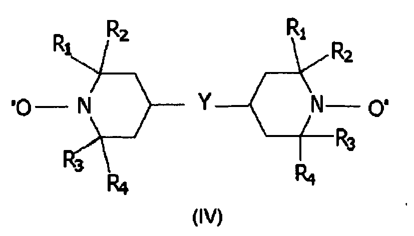 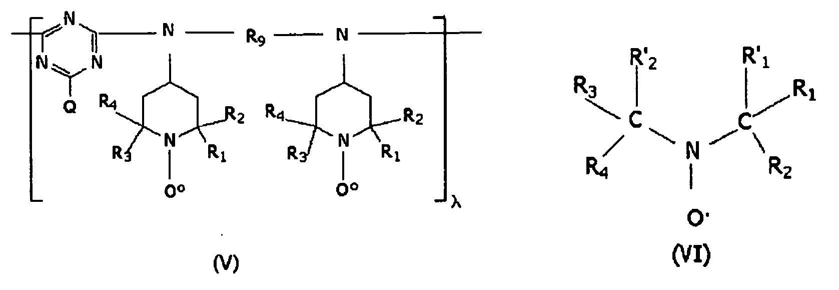 dans lesquelles R₁, R₂, R₃ et R₄ pouvant être identiques ou différents représentent un atome d'hydrogène, un atome d'halogène tel que le fluor, le chlore, le brome ou l'iode, un groupement hydrocarboné linéaire, ramifié ou cyclique, saturé ou insaturé tels qu'un radical alkyle ou phényle, une chaîne de polymère, un groupement fonctionnalisé tel qu'un groupement cyano -CN, un groupement ester-COOR, un groupement amide -CON(R)2, un groupement alcoxyle-OR, un groupement phosphonate -PO(OR)2 avec R représentant une chaîne hydrocarbonée ayant de 1 à 9 atomes de carbone ;
dans lesquelles R₅, R₆, R₇, R₈, pouvant être identiques ou différents, peuvent être choisis dans la même famille de groupement que celle pour R₁. R₂, R₃, R₄ et de plus, peuvent représenter un groupement hydroxyle -OH, un groupement acide tel que -COOH ou --PO(OH)₂ ou -SO₃H ;
X dans la formule (III) représentant :
- un groupement bivalent choisi parmi le méthylène -CH₂ -- , le -C(OR°)(OR')-, le carbonyl - C(O)-, l'oxy -O- et -CHZ- avec Z représentant un reste monovalent choisi parmi les groupements cyano (-CN), hydroxyle (-OH), amino (-NR°R'), alcoxy (-OR°), iminoyl (-N=CR°R'), carboxylate (-OC(O)-R°), amide (-NHR°-C(O)R'), avec R° et R' identiques ou différents représentent un atome d'hydrogène, un groupement alkyle, linéaire ou ramifié ayant un nombre d'atomes de carbone allant de 1 à 20, un groupement benzyle ou phényle, ou
- un groupement phosphonate (-OP(O)R"R''') avec R" et R''' ayant la même signification que Z ;
Y dans la formule (IV) représentant un groupement bivalent choisi parmi :
--OC(O)-(CRₐR_{b})ₙ-C(O)O-- ,
--NH-(CRₐR_{b})ₙ NH-- ,
--NHC(O)-( CRₐR_{b})ₙ --C(O)NH--,
-- S -- , --O-- ;
Rₐ et R_{b} , identiques ou différents, représentant un atome d'hydrogène ou un radical alkyle, linéaire ou ramifié, ayant un nombre d'atomes de carbone allant de 1 à 10 et n représentant un nombre entier allant de 0 à 20 ;
dans la formule (V), λ étant un nombre entier compris entre 1 et 20, R₉ représentant un groupement alkylène ayant un nombre d'atomes de carbone allant de 2 à 12 qui peut être interrompu par un -O-ou -NR₁₀- avec R₁₀ désignant un atome d'hydrogène, un groupement alkyle ayant un nombre d'atomes de carbone compris entre 1 et 12, ou un groupement cycloalkyle et Q représentant un radical --OR₁₁, --NHR₁₂, ou --NR₁₂R₁₃ où R₁₁ représente un radical alkyle, linéaire ou ramifié ayant un nombre d'atomes de carbone allant de 1 à 12, un radical alcoxyalkyle en C₃ - C₁₂, un radical cyclohexyle, un radical benzyle, un radical phényle, un radical tolyle ou un reste 2, 2, 6, 6-tétrapipéridinyle, R₁₂ et R₁₃ ayant la même signification que R_{11,} et de plus, pouvant également former ensemble et avec l'atome d'azote qui les porte un radical hétérocyclique à 5, 6 ou 7 chaînons pouvant contenir en outre un oxygène ;
dans la formule (VI), R'₁ et R'₂ , pouvant être identiques ou différents, ayant la même signification que R₁, R₂, R₃, R₄ des formules précédentes.

3. Utilisation selon la revendication 2 **caractérisée en ce que** le stabilisateur thermique est représenté par la formule suivante : 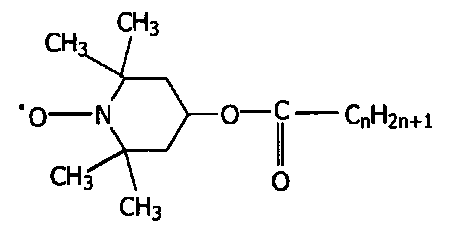 avec n pouvant varier de 1 à 20.

4. Utilisation selon la revendication 2 **caractérisée en ce que** le stabilisateur thermique est choisi parmi le 2,2,6,6-tétraméthyl-1-pipéridinyloxy, le 4-hydroxy 2,2,6,6-tétraméthyl-1-pipéridinyloxy, le 4-méthoxy-2,2,6,6-tétraméthyl-1-pipéridinyloxy, le 4-oxo- 2,2,6,6 tétraméthyl-1-pipéridinyloxy, le 2,2,5,5 tétraméthyl-1-pyrrolidinyloxy, le bis (1-oxyl-2,2,6,6 tétraméthylpipéridine-4-yl) sébacate, le 2,2,6,6-tétraméthyl-4-hydroxypipéridine-1-oxyl monophosphonate, le 3-carboxy-2,2,5,5-tétraméthylpirrolidinyloxy.

## Claims

1. Use of an N-heterocyclic heat-stabilizing compound including at least one nitroxyl radical with the nitrogen atom of the nitroxyl radical forming part of the ring for preparing a thermally stable solution of organic peroxide, the temperature of the beginning of decomposition of the said solution comprising the stabilizer being > the temperature of the beginning of decomposition of the solution not comprising the stabilizer.

2. Use according to Claim 1, **characterized in that** the heat stabilizer is represented by at least one of the following formulae (I), (II), (III), (IV), (V) and (VI): 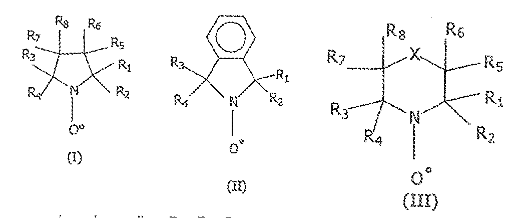 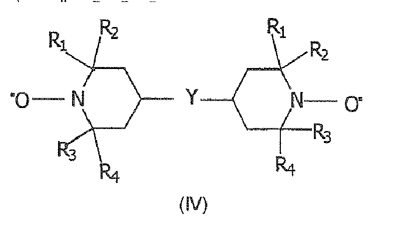 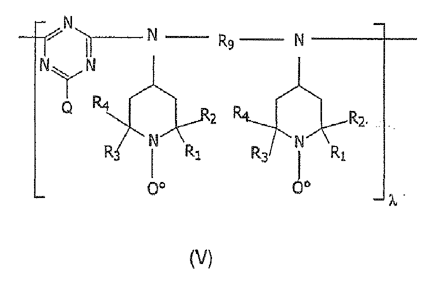 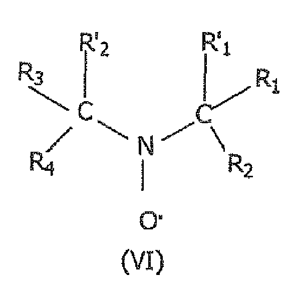 in which R₁, R₂, R₃ and R₄, which can be identical or different, represent a hydrogen atom, a halogen atom, such as fluorine, chlorine, bromine or iodine, a saturated or unsaturated and linear, branched or cyclic hydrocarbon group, such as an alkyl or phenyl radical, a polymer chain or a functional group, such as a cyano group -CN, an ester group -COOR, an amide group -CON(R)₂, an alkoxyl group -OR or a phosphonate group -PO(OR)₂, with R representing a hydrocarbon chain having from 1 to 9 carbon atoms;
in which R₅, R₆, R₇ and R₈, which can be identical or different, can be chosen from the same family of groups as that for R₁, R₂, R₃ and R₄ and, furthermore, can represent a hydroxyl group -OH or an acid group, such as -COOH or -PO(OH)₂ or -SO₃H;
X in the formula (III) representing:
- a divalent group chosen from methylene -CH₂-, -C(OR°)(OR')-, carbonyl -C(O)-, oxy -O- and -CHZ-, with Z representing a monovalent residue chosen from cyano (-CN), hydroxyl (-OH), amino (-NR°R'), alkoxy (-OR°), iminoyl (-N=CR°R'), carboxylate (-OC(O)-R°) or amide (-NHR°-C(O)R') groups and with R° and R', which are identical or different, representing a hydrogen atom, a linear or branched alkyl group having a number of carbon atoms ranging from 1 to 20, or a benzyl or phenyl group, or
- a phosphonate group (-OP(O)R"R)with R"and R having the same meaning as Z;
Y in the formula (IV) representing a divalent group chosen from:
-OC(O)-(CRₐR_{b})ₙ-C(O)O-,
-NH-(CRₐR_{b})ₙNH-
-NHC(O)-(CRₐR_{b})ₙ-C(O)NH-,
-S-, -O-;
Rₐ and R_{b}, which are identical or different, representing a hydrogen atom or a linear or branched alkyl radical having a number of carbon atoms ranging from 1 to 10 and n representing an integer ranging from 0 to 20; in formula (V), λ being an integer between 1 and 20, R₉ representing an alkyl end group having a number of carbon atoms ranging from 2 to 12 which are interrupted by an -O- or -NR₁₀- with R₁₀ denoting a hydrogen atom, an alkyl group having a number of carbon atoms of between 1 and 12 or a cycloalkyl group, and Q representing an -OR₁₁, -NHR₁₂ or -NR₁₂R₁₃ radical where R₁₁ represents a linear or branched alkyl radical having a number of carbon atoms ranging from 1 to 12, a C₃-C₁₂ alkoxyalkyl radical, a cyclohexyl radical, a benzyl radical, a phenyl radical, a tolyl radical or a 2,2,6,6-tetrapiperidinyl radical and R₁₂ and R₁₃ have the same meaning as R₁₁ and, in addition, can also form, together and with the nitrogen atom which carries them, a 5-, 6- or 7-membered heterocyclic radical which can additionally comprise an oxygen;
in the formula (VI), R'₁ and R'₂, which can be identical or different, having the same meaning as R₁, R₂, R₃ and R₄ in the preceding formulae.

3. Use according to Claim 2, **characterized in that** the heat stabilizer is represented by the following formula: 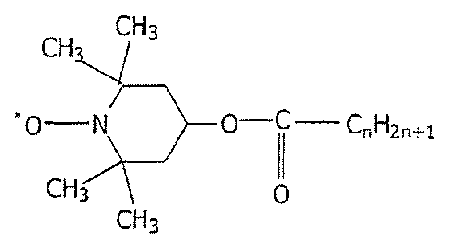 with n being able to vary from 1 to 20.

4. Use according to Claim 2, **characterized in that** the heat stabilizer is chosen from 2,2,6,6-tetramethyl-1-piperidinyloxy, 4-hydroxy-2,2,6,6-tetramethyl-1-piperidinyloxy, 4-methoxy-2,2,6,6-tetramethyl-1-piperidinyloxy, 4-oxo-2,2,6,6-tetramethyl-1-piperidinyloxy, 2,2,5,5-tetramethyl-1-pyrrolidinyloxy, bis(1-oxyl-2,2,6,6-tetramethylpiperidin-4-yl) sebacate, 2,2,6,6-tetramethyl-4-hydroxypiperidine-1-oxyl monophosphonate or 3-carboxy-2,2,5,5-tetramethylpyrrolidinyloxy.

## Patentansprüche

1. Verwendung einer N-heterocyclischen Wärmestabilisatorverbindung mit mindestens einem Nitroxylrest, wobei das Stickstoffatom des Nitroxylrests Teil des Rings ist, zur Herstellung einer wärmestabilen Lösung von organischem Peroxid, wobei die Temperatur des Zersetzungsbeginns der den Stabilisator enthaltenden Lösung größer ist als die Temperatur des Zersetzungsbeginns der Lösung ohne den Stabilisator.

2. Verwendung nach Anspruch 1, **dadurch gekennzeichnet, daß** der Wärmestabilisator durch mindestens eine der folgenden Formeln (I), (II), (III), (IV), (V) und (VI) wiedergegeben wird: 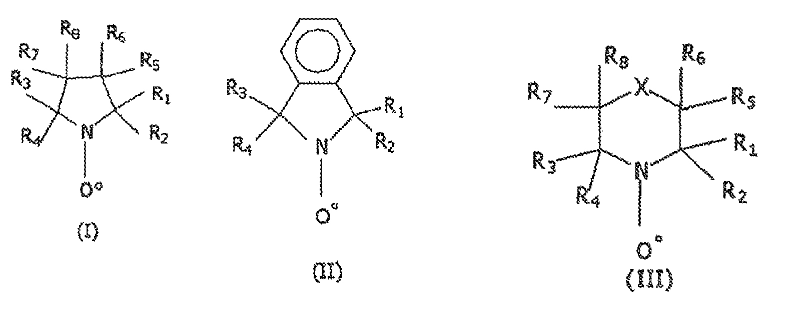 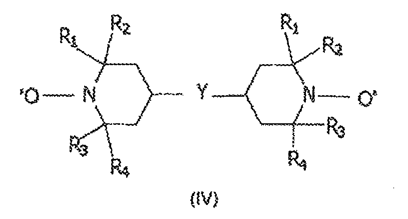 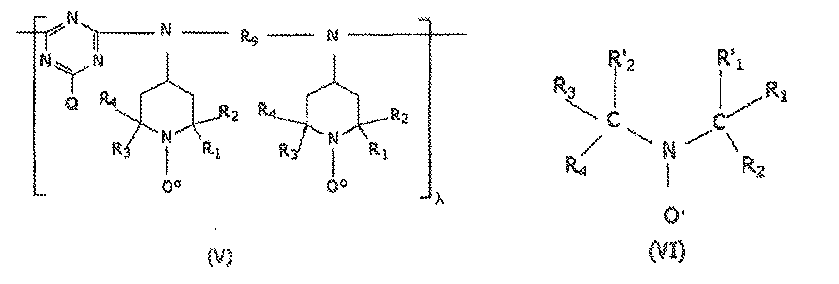 worin R₁, R₂, R₃ und R₄ gleich oder verschieden sein können und für ein Wasserstoffatom, ein Halogenatom wie Fluor, Chlor, Brom oder Iod, eine gesättigte oder ungesättigte, lineare, verzweigte oder cyclische Kohlenwasserstoffgruppe wie ein Alkyl- oder Phenylrest, eine Polymerkette oder eine funktionelle Gruppe wie eine Cyanogruppe -CN, eine Estergruppe -COOR, eine Amidgruppe CON(R)₂, eine Alkoxygruppe -OR oder eine Phosphonatgruppe -PO(OR)₂, worin R eine Kohlenwasserstoffkette mit 1 bis 9 Kohlenstoffatomen bedeutet, stehen;
worin R₅, R₆, R₇ und R₈ gleich oder verschieden sein können und aus der gleichen Gruppenfamilie wie R₁, R₂, R₃ und R₄ ausgewählt sein können und außerdem für eine Hydroxylgruppe -OH oder eine Säuregruppe wie -COOH oder -PO(OH)₂ oder -SO₃H stehen können;
X in Formel (III) für:
eine unter Methylen -CH₂-, -C(OR°)(OR')-, Carbonyl -C(O)-, Oxy -O-und -CHZ-, wobei Z einen unter Cyano- (-CN), Hydroxyl- (-OH), Amino- (-NR°R'), Alkoxy- (-OR°), Iminoyl- (-N=CR°R'), Carboxylat-(-OC(O)-R°) oder Amidgruppen (-NHR°-C(O)R') ausgewählten einwertigen Rest bedeutet und R° und R' gleich oder verschieden sind und ein Wasserstoffatom, eine lineare oder verzweigte Alkylgruppe mit 1 bis 20 Kohlenstoffatomen oder eine Benzyl oder Phenylgruppe bedeuten, ausgewählte Gruppe oder
eine Phosphonatgruppe (-OP(O)R"R"'), wobei R" und R''' die gleiche Bedeutung wie Z besitzen,
steht;
Y in Formel (IV) für eine unter
-OC(O)-(CRₐR_{b})ₙ-C(O)O-,
-NH-(CRₐR_{b})ₙNH-,
-NHC(O)-(CRₐR_{b})ₙ-C(O)NH-,
-S- und -O-
ausgewählte zweiwertige Gruppe steht;
Rₐ und R_{b} gleich oder verschieden sind und für ein Wasserstoffatom oder einen linearen oder verzweigten Alkylrest mit 1 bis 10 Kohlenstoffatomen stehen und n für eine ganze Zahl von 0 bis 20 steht;
in Formel (V) λ für eine ganze Zahl zwischen 1 und 20 steht, R₉ für eine Alkylgruppe mit 2 bis 12 Kohlenstoffatomen steht, die durch - O- oder -NR₁₀-, worin R₁₀ ein Wasserstoffatom, eine Alkylgruppe mit zwischen 1 und 12 Kohlenstoffatomen oder eine Cycloalkylgruppe bedeutet, unterbrochen sein kann, und Q für einen -OR₁₁-, -NHR₁₂-oder NR₁₂R₁₃-Rest, worin R₁₁ einen linearen oder verzweigten Alkylrest mit 1 bis 12 Kohlenstoffatomen, einen C₃-C₁₂-Alkoxyalkylrest, einen Benzylrest, einen Phenylrest, einen Tolylrest oder einen 2,2,6,6-Tetrapiperidinylrest bedeutet, R₁₂ und R₁₃ die gleiche Bedeutung wie R₁₁ besitzen und außerdem gemeinsam mit dem Stickstoffatom, das sie trägt, einen 5-, 6- oder 7-gliedrigen heterocyclischen Rest, der außerdem ein Sauerstoffatom enthalten kann, bilden können, steht;
in Formel (VI) R'₁ und R'₂ gleich oder verschieden sein können und
die gleiche Bedeutung wie R₁, R₂, R₃ und R₄ in den vorhergehenden Formeln besitzen.

3. Verwendung nach Anspruch 2, **dadurch gekennzeichnet, daß** der Wärmestabilisator der folgenden Formel entspricht: 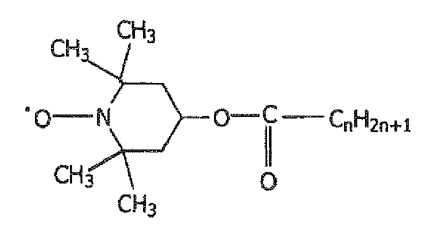 wobei n von 1 bis 20 variieren kann.

4. Verwendung nach Anspruch 2, **dadurch gekennzeichnet, daß** der Wärmestabilisator unter 2,2,6,6-Tetramethyl-1-piperidinyloxy, 4-Hydroxy-2,2,6,6-tetramethyl-1-piperidinyloxy, 4-Methoxy-2,2,6,6-tetramethyl-1-piperidinyloxy, 4-Oxo-2,2,6,6-tetramethyl-1-piperidinyloxy, 2,2,5,5-Tetramethyl-1-pyrrolidinyloxy, Bis(1-oxyl-2,2,6,6-tetramethylpiperidin-4-yl)sebacat, 2,2,6,6-Tetramethyl-4-hydroxypiperidin-1-oxylmonophosphonat oder 3-Carboxy-2,2,5,5-tetramethylpyrrolidinyloxy ausgewählt ist.
